(19) Europäisches Patentamt
European Patent Office
Office européen des brevets

(11) Veröffentlichungsnummer: **0 548 711 A1**

(12) **EUROPÄISCHE PATENTANMELDUNG**

(21) Anmeldenummer: **92121143.9**

(22) Anmeldetag: **11.12.92**

(51) Int. Cl.5: **A01N 37/38**, A01N 37/40, A01N 41/10, A01N 43/16, A01N 43/30

(30) Priorität: **21.12.91 DE 4142514**

(43) Veröffentlichungstag der Anmeldung: **30.06.93 Patentblatt 93/26**

(84) Benannte Vertragsstaaten: **AT BE CH DE DK ES FR GB GR IE IT LI NL PT SE**

(71) Anmelder: **BASF Aktiengesellschaft**
**Carl-Bosch-Strasse 38**
**W-6700 Ludwigshafen(DE)**

(72) Erfinder: **Müller, Thomas, Dr.**
**Bergstrasse 19**
**W-6717 Hessheim(DE)**
Erfinder: **Janssen, Bernd, Dr.**
**Leuschnerstrasse 18a**
**W-6700 Ludwigshafen(DE)**
Erfinder: **Zierke, Thomas, Dr.**
**Akazienstrasse 12**
**W-6737 Boehl-Iggelheim(DE)**
Erfinder: **Eicken, Karl, Dr.**
**Am Hüttenwingert 12**
**W-6706 Wachenheim(DE)**
Erfinder: **Ammermann, Eberhard, Dr.**
**Von-Gagern-Strasse 2**
**W-6148 Heppenheim(DE)**
Erfinder: **Lorenz, Gisela, Dr.**
**Erlenweg 13**
**W-6730 Neustadt(DE)**

(54) **Verfahren zur Bekämpfung von Pilzen.**

(57) Verfahren zur Bekämpfung von Pilzen, wobei man eine fungizid wirksame Menge eines Diphenylheteroalkyl-Derivates der Formel I

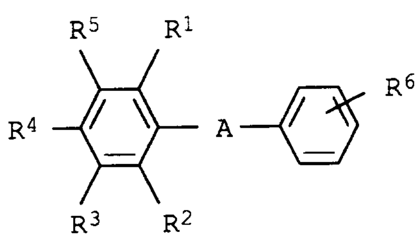

I

in der die Substituenten die folgenden Bedeutungen haben

A eine $CH_2$ und O oder S enthaltende Gruppe

$R^1,R^2,R^3,R^4,R^5$ unabhängig voneinander Wasserstoff, Halogen, eine Alkyl-, eine Phenyl-, eine Halogenalkyl- oder eine $OR^7$-Gruppe

oder

$R^2$ und $R^3$ gemeinsam unter Ausbildung eines Ringes eine -CH = CH-CH = CH-Gruppe

oder

$R^4$ und $R^5$ gemeinsam unter Ausbildung eines Ringes eine $-CH_2-CH_2-CH_2-CH_2$-Gruppe, eine $-CH_2-CH_2-CH_2$-Gruppe, eine -CH = CH-CH = CH-Gruppe oder eine $-O-CH_2-O$-Gruppe,

$R^6$ eine $COOR^{11}$-Gruppe, die in o-, m- oder p-Position zu A stehen kann,

$R^{11}$ Wasserstoff oder Alkylgruppe bedeuten,

EP 0 548 711 A1

oder dessen pflanzenverträglichen Salzes auf Pilze, vom Pilzbefall bedrohte Pflanzen, deren Lebensraum, deren Saatgut oder auf Materialien einwirken läßt.

Die vorliegende Erfindung betrifft ein Verfahren zur Bekämpfung von Pilzen mit Diphenylheteroalkyl-Derivaten.

Es ist bekannt, daß einige Diphenylheteroalkyl-Verbindungen pharmakologische (vgl. DE 24 39 458 und EP 386 452), wachstumsregulatorische (vgl. DE 22 52 818) oder herbizide (vgl. C.A. 98:71709x) Wirkungen besitzen. Es ist ferner bekannt, daß 2-Cyano-N-(ethylaminocarbonyl)-2-methoximiho-acetamid fungizid wirksam ist (US-3 957 847).

Überraschend wurde nun gefunden, daß Diphenylheteroalkyl-Derivate der Formel I

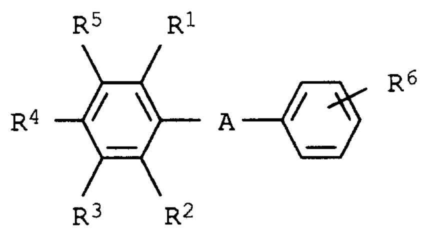

I

in der die Substituenten die folgenden Bedeutungen haben

| | |
|---|---|
| A | eine $-X-CH_2-$, eine $-X-CH(CH_3)-$, eine $-CH_2-X-$oder eine $-CH(CH_3)-X-$Gruppe, wobei X für ein Sauerstoffatom oder eine $S(O)_n$-Gruppe steht (mit n in der Bedeutung von 0, 1 oder 2) |
| $R^1$,$R^2$,$R^3$,$R^4$,$R^5$ | unabhängig voneinander Wasserstoff oder Halogen, eine $C_1$-$C_{12}$-Alkyl-, eine gegebenenfalls substituierte Phenyl-, eine $C_1$-$C_3$-Halogenalkyl- oder eine $OR^7$-Gruppe mit der Maßgabe, daß nur vier der Substituenten $R^1$, $R^2$, $R^3$, $R^4$ und $R^5$ Wasserstoff sein können |

oder

| | |
|---|---|
| $R^2$ und $R^3$ | gemeinsam unter Ausbildung eines Ringes eine $-CH=CH-CH=CH-$Gruppe |

oder

| | |
|---|---|
| $R^4$ und $R^5$ | gemeinsam unter Ausbildung eines Ringes eine $-CH_2-B-CH_2-$Gruppe [mit B in der Bedeutung $-CH_2-$ oder $-(CH_2)_2-$], eine $-C(CH_3)(CH_2R^8)-D-C(CH_3)_2-$Gruppe [mit D in der Bedeutung von $-(CH_2)_2-$, $-CH_2-CH(CH_3)-$ oder $-CHR^9-$], eine $-O-C(CH_3)(Z)-CH_2-CH_2-$Gruppe [mit Z = Methyl oder Ethyl, die jeweils durch den Rest $OR^{10}$ substituiert sein können], eine $-CH=CH-CH=CH-$Gruppe oder eine $-O-CH_2-O-$Gruppe, |
| $R^6$ | eine $COOR^{11}$-Gruppe, die in o-, m- oder p-Position zu A stehen kann, |
| $R^7$ | Wasserstoff, eine $C_1$-$C_{12}$-Alkyl-, eine $C_3$-$C_6$-Alkenyl-, eine $C_3$-$C_6$-Alkinyl-, eine $C_1$-$C_6$-Alkanoyl-, eine gegebenenfalls substituierte Benzyl- oder eine gegebenenfalls substituierte Phenylgruppe, |
| $R^8$ | Wasserstoff oder eine $C_1$-$C_6$-Alkylgruppe, |
| $R^9$ | Wasserstoff oder eine $C_1$-$C_4$-Alkylgruppe, |
| $R^{10}$ | Wasserstoff, eine $C_1$-$C_6$-Alkyl- oder eine $C_1$-$C_6$-Alkanoylgruppe, |
| $R^{11}$ | Wasserstoff oder eine $C_1$-$C_4$-Alkylgruppe, |

sowie ihre pflanzenverträglichen Salze eine ausgezeichnete fungizide Wirkung gegen phytopathogene Pilze besitzen.

Im Hinblick auf ihre fungizide Wirkung werden diejenigen Verbindungen der Formel I bevorzugt, in denen

$R^1$,$R^2$,$R^3$,$R^4$,$R^5$ Wasserstoff, mit der Maßgabe, daß mindestens einer der Substituenten verschieden von Wasserstoff ist;

Halogen, insbesondere Fluor und Chlor;

$C_1$-$C_{12}$-Alkyl, insbesondere Methyl, Ethyl, Propyl, 1-Methylethyl, Butyl, 1-Methylpropyl, 2-Methylpropyl, 1,1-Dimethylethyl, n-Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, n-Heptyl, 1-Methylhexyl, 2-Methylhexyl, 3-Methylhexyl, 4-Methylhexyl, 5-Methylhexyl, 1-Ethylpentyl, 2-Ethylpentyl, 1-Propylbutyl, Octyl und 1,1,3,3-Tetramethylbutyl;

Phenyl;
$C_1$-$C_3$-Halogenalkyl, insbesondere Trifluormethyl;
Hydroxy;
$C_1$-$C_{12}$-Alkoxy, insbesondere Methoxy, Ethoxy, Propyloxy, 1-Methylethoxy, Butyloxy, 1-Methylpropyloxy, 2-Methylpropyloxy, 1,1-Dimethylethoxy, n-Pentyloxy, Octyloxy und Decyloxy;
$C_1$-$C_6$-Alkanoyloxy, insbesondere Methylcarbonyloxy (Acetyloxy, $CH_3COO$-) Ethylcarbonyloxy und 1,1-Dimethylethylcarbonyloxy;
Benzyloxy;
Phenoxy, wobei diese Gruppe bis zu drei Halogenatome, insbesondere Fluor und Chlor, tragen kann;

$R^2$ und $R^3$ gemeinsam unter Ausbildung eines Ringes eine -CH = CH-CH = CH-Gruppe;
R4 und R5 gemeinsam unter Ausbildung eines Ringes eine $-CH_2$-B-$CH_2$-Gruppe [mit B in der Bedeutung $-CH_2$- oder $-(CH_2)_2$-],
eine $-C(CH_3)(CH_2R^8)$-D-$C(CH_3)_2$-Gruppe [mit D in der Bedeutung von $-(CH_2)_2$-, $-CH_2$-$CH(CH_3)$- oder $-CHR^9$-], wobei $R^8$ für ein Wasserstoffatom oder $C_1$-$C_6$-Alkyl, insbesondere Methyl, Ethyl, Propyl, 1-Methylethyl, Butyl, 1-Methylpropyl, 2-Methylpropyl, 1,1-Dimethylethyl und n-Pentyl steht und $R^9$ für ein Wasserstoffatom oder $C_1$-$C_4$-Alkyl, insbesondere Methyl und Ethyl, steht,
eine $-O-C(CH_3)(Z)-CH_2-CH_2$-Gruppe [mit Z = Methyl, das mit Hydroxy substituiert sein kann],
eine -CH = CH-CH = CH-Gruppe oder
ein $-O-CH_2-O$-Gruppe;
$R^{11}$ Wasserstoff und
X Sauerstoff

bedeuten und $R^6$ in ortho oder para-Position zu A steht.

Diphenylheteroalkyl-Derivate der Formel I mit $R^6$ = COOH können mit Basen in üblicher Weise in pflanzenverträgliche, wasserlösliche Salze übergeführt werden. Geeignete Salze sind beispielsweise Ammonium- und Alkalimetallsalze, insbesondere des Natriums, Kaliums und Lithiums, oder Erdalkalimetallsalze, insbesondere des Calciums oder Magnesiums, sowie Salze mit geeigneten organischen Basen, wie mit niederen Aklylaminen, z.B. Methylamin, Ethylamin oder Cyclohexylamin, oder mit substituierten niederen Alkylaminen, insbesondere hydroxysubstituierten Alkylaminen, wie Diethanolamin, Triethanolamin oder Tris-(hydroxymethyl)aminomethan sowie mit Piperidin oder Morpholin.

Die Verbindungen der Formel I enthalten teilweise chirale Zentren und fallen im allgemeinen als Diastereomerengemische bzw. Racemate an. Die so erhaltenen Diastereomeren lassen sich beispielsweise durch Löslichkeitsunterschiede oder durch Säulenchromatographie trennen und in reiner Form isolieren. Aus den Enantiomerenpaaren kann man nach bekannten Methoden einheitliche Enantiomere erhalten. Sowohl diese als auch ihre Gemische (Racemate) sind für die vorliegende Erfindung geeignet. Zur Bekämpfung von phytopathogenen Pilzerkrankungen kann man sowohl die einheitlichen Diastereomeren bzw. Enantiomeren wie auch deren Gemische verwenden.

Die Diphenylheteroalkyl-Derivate I sind teilweise bekannt oder können nach bekannten Methoden dargestellt werden (vgl. EP 386 452, DE 24 39 458, DE 22 52 818 oder C.A. 98:71709x).

Beispielsweise kann die Herstellung durch Umsetzung der Verbindung II mit der Verbindung III in Gegenwart von Alkali erfolgen.

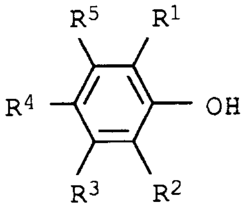

II

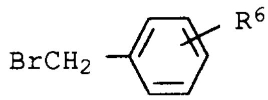

III

Beispielsweise ist auch die Umsetzung der Verbindungen IV und V in Gegenwart von Alkali möglich.

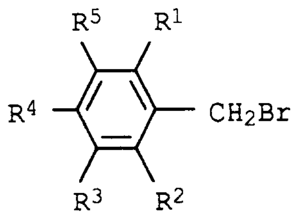

IV

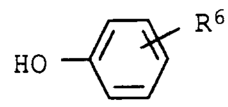

V

Die Verbindungen der Formel I und ihre pflanzenverträglichen Salze eignen sich als Fungizide, insbesondere gegenüber Phycomyceten, bei guter Pflanzenverträglichkeit.

Beispiele

Herstellungsbeispiel:
(4-Carboxybenzyl)-(2-methyl-4-t-butyl-phenyl)ether (Verbindung 16a)

Zu einer Suspension von 1,44 g (60 mmol) NaH in 50 ml DMF (Dimethylformamid) wird unter $N_2$-Atmosphäre eine Lösung von 8,2 g (50 mmol) 2-Methyl-4-t-butyl-phenol in 50 ml DMF bei Raumtemperatur zugetropft und man läßt 40 Minuten nachrühren. Danach tropf man eine Lösung von 13,3 g (50 mmol) 4-Carbethoxybenzylbromid in 50 ml DMF bei Raumtemperatur zu und läßt weitere 60 Minuten nachrühren. Der Reaktionsansatz wird auf 300 ml einer Eis-Kochsalz-Mischung gegossen und mit Diethylether extrahiert. Nach Trocknen der vereinigten Etherphasen über $MgSO_4$ und Abdestillieren des Lösungsmittels im Vakuum bleiben 16,3 g eines farblosen Festkörpers zurück. Der Feststoff wird zusammen mit 80 ml Ethanol, 10 ml DMSO (Dimethylsulfoxyd), 40 ml Wasser und 6 g KOH 30 Minuten unter Rückfluß erhitzt. Anschließend gießt man den Reaktionsansatz auf eine Eis-Kochsalz-Mischung und säuert mit konzentrierter HCl an. Der ausgefallene farblose Festkörper wird abgesaugt, mit Wasser säurefrei gewaschen und getrocknet.

Ausbeute: 8,5 g; Schmelzpunkt: 193 - 198 °C.

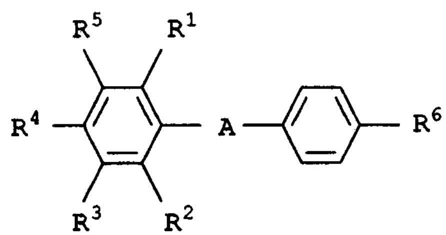

Ia

Me = Methyl,
Et = Ethyl

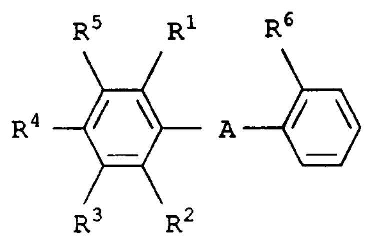

Ib

Me = Methyl,
Et = Ethyl

## Verfahren zur Bekämpfung von Pilzen

### Tabellen

### Tabelle 1a

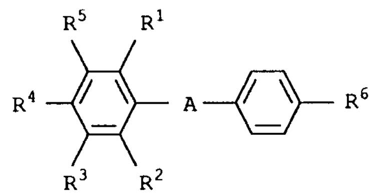

Ia

Me = Methyl,
Et = Ethyl

| Bsp.-Nr. | $R^1$ | $R^2$ | $R^3$ | $R^4$ | $R^5$ | $R^6$ | A | Schmp. (°C) bzw. $\gamma_{CO}$ ($cm^{-1}$) |
|---|---|---|---|---|---|---|---|---|
| 1a | H | H | H | $-C(CH_3)_2CH_2CH_2C(CH_3)_2-$ | | $CO_2H$ | $-OCH_2-$ | > 330 |
| 2a | H | H | H | $-C(CH_3)_2CH_2CH_2C(CH_3)_2-$ | | $CO_2Et$ | $-OCH_2-$ | 76 - 77 |
| 3a | H | H | H | $-C(CH_3)_2CH_2CH_2C(CH_3)_2-$ | | $CO_2Et$ | $-CH_2O-$ | 109 - 111 |
| 4a | H | H | H | $-C(CH_3)_2CH_2CH_2C(CH_3)_2-$ | | $CO_2H$ | $-CH_2O-$ | 185 - 186 |
| 5a | H | H | H | $-C(CH_3)_2CH_2CH_2C(CH_3)_2-$ | | $CO_2H$ | $-SCH_2-$ | 169 - 170 |
| 6a | H | H | H | $-C(CH_3)_2CH_2CH_2C(CH_3)_2-$ | | $CO_2H$ | $-SOCH_2-$ | 198 - 200 |
| 7a | H | H | H | $-C(CH_3)_2CH_2CH_2C(CH_3)_2-$ | | $CO_2H$ | $-SO_2CH_2-$ | 202 - 205 |
| 8a | H | H | H | $-C(CH_3)_2CH_2CH_2\ C(CH_3)_2-$ | | $CO_2H$ | $-CH_2S-$ | 197 - 199 |
| 9a | H | H | H | $-C(CH_3)_2CH_2CH_2\ C(CH_3)_2-$ | | $CO_2H$ | $-CH(CH_3)S-$ | 172 - 175 |
| 10a | H | H | H | $-C(CH_3)_2CH_2CH_2\ C(CH_3)_2-$ | | $CO_2H$ | $-SCH(CH_3)-$ | 138 - 140 |
| 11a | OH | H | OH | $-C(CH_3)_2CH_2CH_2\ C(CH_3)_2-$ | | $CO_2H$ | $-OCH_2-$ | 190 - 196 |
| 12a | H | H | $C(CH_3)_3$ | H | $C(CH_3)_3$ | $CO_2H$ | $-OCH_2-$ | 198 - 207 |
| 13a | H | H | H | $C(CH_3)_3$ | H | $CO_2H$ | $-OCH_2-$ | 233 - 242 |

| Bsp.-Nr. | $R^1$ | $R^2$ | $R^3$ | $R^4$ | $R^5$ | $R^6$ | A | Schmp. (°C) bzw. $\Upsilon_{CO}$ ($cm^{-1}$) |
|---|---|---|---|---|---|---|---|---|
| 14a | H | H | H | $C(CH_3)_2C_2H_5$ | H | $CO_2H$ | $-OCH_2-$ | 208 - 213 |
| 15a | $C(CH_3)_3$ | H | $C(CH_3)_3$ | H | H | $CO_2H$ | $-OCH_2-$ | 230 - 235 |
| 16a | H | $CH_3$ | H | $C(CH_3)_3$ | H | $CO_2H$ | $-OCH_2-$ | 193 - 198 |
| 17a | $C(CH_3)_3$ | H | $CH_3$ | $C(CH_3)_3$ | H | $CO_2H$ | $-OCH_2-$ | 235 - 238 |
| 18a | $C(CH_3)_3$ | H | H | $CH_3$ | H | $CO_2H$ | $-OCH_2-$ | 210 - 212 |
| 19a | H | H | $C(CH_3)_3$ | H | H | $CO_2H$ | $-OCH_2-$ | 181 - 184 |
| 20a | H | $CH(CH_3)C_2H_5$ | H | $C(CH_3)_3$ | H | $CO_2H$ | $-OCH_2-$ | 208 - 215 |
| 21a | $C(CH_3)_3$ | $CH(CH_3)_2$ | H | $C(CH_3)_3$ | H | $CO_2H$ | $-OCH_2-$ | 202 - 210 |
| 22a | H | OH | $C(CH_3)_3$ | H | $C(CH_3)_3$ | $CO_2H$ | $-OCH_2-$ | 210 - 212 |
| 23a | $C(CH_3)_3$ | H | H | $C(CH_3)_3$ | H | $CO_2H$ | $-OCH_2-$ | 248 - 250 |
| 24a | $OCOCH_3$ | H | $OCOCH_3$ | $-C(CH_3)_2CH_2CH\ _3C(CH_3)_3$ | | $CO_2H$ | $-OCH_2-$ | 164 - 168 |
| 25a | $CH_3$ | $CH_3$ | $CH_3$ | $-OC(CH_3)_2CH_2-CH_2-$ | | $CO_2H$ | $-OCH_2-$ | 193 - 196 |
| 26a | $CH_3$ | $CH_3$ | $CH_3$ | $-OC(CH_3)(C_2H_4OH)CH_2CH_2-$ | | $CO_2H$ | $-OCH_2-$ | 179 - 183 |
| 27a | H | H | $C(CH_3)_3$ | H | $C(CH_3)_3$ | $CO_2H$ | $-SCH_2-$ | 149 - 152 |
| 28a | $CH_3$ | $CH_3$ | $CH_3$ | $-OC(CH_3)_2-CH_2CH_2-$ | | $CO_2Et$ | $-OCH_2-$ | 76 - 77 |
| 29a | H | H | H | $C(CH_3)_2C_2H_5$ | H | $CO_2Me$ | $-OCH_2-$ | 51 - 54 |
| 30a | $CH_3$ | H | H | H | H | $CO_2Et$ | $-OCH_2-$ | 70 |
| 31a | H | H | $CH_3$ | H | H | $CO_2Et$ | $-OCH_2-$ | 34 |
| 32a | H | H | H | $CH_3$ | H | $CO_2Et$ | $-OCH_2-$ | 47 |
| 33a | $CH_3$ | H | H | H | $CH_3$ | $CO_2Et$ | $-OCH_2-$ | 61 - 65 |
| 34a | $CH_3$ | H | H | $CH_3$ | H | $CO_2Et$ | $-OCH_2-$ | 1725 |
| 35a | $CH_3$ | $CH_3$ | H | H | H | $CO_2Et$ | $-OCH_2-$ | 1719 |
| 36a | H | H | $CH_3$ | $CH_3$ | H | $CO_2Et$ | $-OCH_2-$ | 58 - 60 |

| Bsp.-Nr. | $R^1$ | $R^2$ | $R^3$ | $R^4$ | $R^5$ | $R^6$ | A | Schmp. (°C) bzw. $\nu_{CO}$ ($cm^{-1}$) |
|---|---|---|---|---|---|---|---|---|
| 37a | $CH_3$ | H | $CH_3$ | H | $CH_3$ | $CO_2Et$ | $-OCH_2-$ | 1720 |
| 38a | $CH_3$ | $CH_3$ | $CH_3$ | H | H | $CO_2Et$ | $-OCH_2-$ | 1722 |
| 39a | H | H | H | -CH=CH-CH=CH- | | $CO_2Et$ | $-OCH_2-$ | 70 - 72 |
| 40a | Cl | H | H | H | H | $CO_2Et$ | $-OCH_2-$ | 71 |
| 41a | H | H | Cl | H | H | $CO_2Et$ | $-OCH_2-$ | 1722 |
| 42a | H | H | H | Cl | H | $CO_2Et$ | $-OCH_2-$ | 64 |
| 43a | Cl | H | H | H | Cl | $CO_2Et$ | $-OCH_2-$ | 94 - 98 |
| 44a | Cl | H | H | Cl | H | $CO_2Et$ | $-OCH_2-$ | 79 |
| 45a | Cl | H | Cl | H | H | $CO_2Et$ | $-OCH_2-$ | 87 - 92 |
| 46a | H | H | Cl | Cl | H | $CO_2Et$ | $-OCH_2-$ | 1715 |
| 47a | H | H | Cl | H | Cl | $CO_2Et$ | $-OCH_2-$ | 63 - 72 |
| 48a | H | H | H | F | H | $CO_2Et$ | $-OCH_2-$ | 50 |
| 49a | F | H | H | F | H | $CO_2Et$ | $-OCH_2-$ | 54 - 57 |
| 50a | F | F | F | F | F | $CO_2Et$ | $-OCH_2-$ | 46 - 51 |
| 51a | $CH_3$ | H | H | H | H | $CO_2H$ | $-OCH_2-$ | 174 - 175 |
| 52a | H | H | $CH_3$ | H | H | $CO_2H$ | $-OCH_2-$ | 154 - 198 |
| 53a | H | H | H | $CH_3$ | H | $CO_2H$ | $-OCH_2-$ | 188 - 190 |
| 54a | $CH_3$ | H | H | H | $CH_3$ | $CO_2H$ | $-OCH_2-$ | 186 - 190 |
| 55a | $CH_3$ | H | H | $CH_3$ | H | $CO_2H$ | $-OCH_2-$ | 170 - 180 |
| 56a | $CH_3$ | $CH_3$ | H | H | H | $CO_2H$ | $-OCH_2-$ | 135 - 145 |
| 57a | H | H | $CH_3$ | $CH_3$ | H | $CO_2H$ | $-OCH_2-$ | 204 - 206 |
| 58a | $CH_3$ | H | $CH_3$ | H | $CH_3$ | $CO_2H$ | $-OCH_2-$ | 179 - 188 |
| 59a | $CH_3$ | $CH_3$ | $CH_3$ | H | H | $CO_2H$ | $-OCH_2-$ | 136 - 153 |

| Bsp.-Nr. | $R^1$ | $R^2$ | $R^3$ | $R^4$ | $R^5$ | $R^6$ | A | Schmp. (°C) bzw. $\Upsilon_{CO}$ ($cm^{-1}$) |
|---|---|---|---|---|---|---|---|---|
| 60a | H | H | H | $-CH=CH-CH=CH-$ | | $CO_2H$ | $-OCH_2-$ | 1685 |
| 61a | Cl | H | H | H | H | $CO_2H$ | $-OCH_2-$ | 189 - 195 |
| 62a | H | H | Cl | H | H | $CO_2H$ | $-OCH_2-$ | 148 - 152 |
| 63a | H | H | H | Cl | H | $CO_2H$ | $-OCH_2-$ | 190 - 202 |
| 64a | Cl | H | H | H | Cl | $CO_2H$ | $-OCH_2-$ | 201 - 202 |
| 65a | Cl | H | Cl | H | H | $CO_2H$ | $-OCH_2-$ | 173 - 250 |
| 66a | H | H | Cl | Cl | H | $CO_2H$ | $-OCH_2-$ | 175 - 250 |
| 67a | H | H | Cl | H | Cl | $CO_2H$ | $-OCH_2-$ | 151 - 193 |
| 68a | H | H | H | F | H | $CO_2H$ | $-OCH_2-$ | 200 - 210 |
| 69a | F | H | H | F | H | $CO_2H$ | $-OCH_2-$ | 1688 |
| 70a | F | F | F | F | F | $CO_2H$ | $-OCH_2-$ | 138 - 150 |
| 71a | Cl | H | H | Cl | H | $CO_2H$ | $-OCH_2-$ | 205 - 207 |
| 72a | $CH_3$ | H | $CH_3$ | H | H | $CO_2Me$ | $-OCH_2-$ | 70 - 75 |
| 73a | H | H | $CH_3$ | H | $CH_3$ | $CO_2Me$ | $-OCH_2-$ | 52 - 57 |
| 74a | $CH_3$ | $CH_3$ | H | $CH_3$ | H | $CO_2Me$ | $-OCH_2-$ | 1720 |
| 75a | $CH_3$ | H | H | $C(CH_3)_2CH_2C(CH_3)_3$ | H | $CO_2Me$ | $-OCH_2-$ | 71 - 74 |
| 76a | H | H | H | $C(CH_3)_2CH_2C(CH_3)_3$ | H | $CO_2Me$ | $-OCH_2-$ | 77 - 82 |
| 77a | $CH(CH_3)_2$ | H | H | H | H | $CO_2Me$ | $-OCH_2-$ | 74 - 80 |
| 78a | H | H | H | $-CH_2-CH_2-CH_2-$ | | $CO_2Me$ | $-OCH_2-$ | 84 - 87 |
| 79a | H | H | H | $-CH_2-CH_2-CH_2-CH_2-$ | | $CO_2Me$ | $-OCH_2-$ | 50 - 55 |
| 80a | H | H | Ph | H | H | $CO_2Me$ | $-OCH_2-$ | 103 - 111 |
| 81a | H | H | $CH_3$ | H | $CH_3$ | $CO_2H$ | $-OCH_2-$ | 170 - 173 |
| 82a | $CH_3$ | H | $CH_3$ | H | H | $CO_2H$ | $-OCH_2-$ | 154 - 165 |

| Bsp.-Nr. | $R^1$ | $R^2$ | $R^3$ | $R^4$ | $R^5$ | $R^6$ | A | Schmp. (°C) bzw. $\gamma_{CO}$ ($cm^{-1}$) |
|---|---|---|---|---|---|---|---|---|
| 83a | $CH_3$ | $CH_3$ | H | $CH_3$ | H | $CO_2H$ | $-OCH_2-$ | 174 - 181 |
| 84a | $CH_3$ | H | H | $C(CH_3)_2CH_2C(CH_3)_3$ | H | $CO_2H$ | $-OCH_2-$ | 151 - 179 |
| 85a | H | H | H | $C(CH_3)_2CH_2C(CH_3)_3$ | H | $CO_2H$ | $-OCH_2-$ | 210 - 212 |
| 86a | $CH(CH_3)_2$ | H | H | H | H | $CO_2H$ | $-OCH_2-$ | 150 - 155 |
| 87a | H | H | $CH(CH_3)_2$ | H | H | $CO_2H$ | $-OCH_2-$ | 1690 |
| 88a | H | H | H | $CH(CH_3)_2$ | H | $CO_2H$ | $-OCH_2-$ | 1688 |
| 89a | H | H | H | $-CH_2-CH_2-CH_2-$ | | $CO_2H$ | $-OCH_2-$ | 194 - 232 |
| 90a | H | H | H | $-CH_2-CH_2-CH_2-CH_2-$ | | $CO_2H$ | $-OCH_2-$ | 180 - 183 |
| 91a | H | H | Ph | H | H | $CO_2H$ | $-OCH_2-$ | 1683 |
| 92a | OMe | H | H | H | H | $CO_2H$ | $-OCH_2-$ | 179 - 186 |
| 93a | H | H | OMe | H | H | $CO_2H$ | $-OCH_2-$ | 147 - 210 |
| 94a | H | H | OMe | OMe | H | $CO_2H$ | $-OCH_2-$ | 144 - 165 |
| 95a | H | H | OMe | H | OMe | $CO_2H$ | $-OCH_2-$ | 154 - 161 |
| 96a | OMe | OMe | H | H | H | $CH_2H$ | $-OCH_2-$ | 132 - 142 |
| 97a | H | H | OMe | OMe | OMe | $CO_2H$ | $-OCH_2-$ | 174 - 179 |
| 98a | $OCH(CH_3)_2$ | H | H | H | H | $CO_2H$ | $-OCH_2-$ | 107 - 112 |
| 99a | H | H | OPh | H | H | $CO_2H$ | $-OCH_2-$ | 174 - 219 |
| 100a | H | H | H | $O-2,4-Cl_2C_6H_3$ | H | $CO_2H$ | $-OCH_2-$ | 1690 |
| 101a | H | H | $OCH_2Ph$ | H | H | $CO_2H$ | $-OCH_2-$ | 129 - 135 |
| 102a | H | H | H | $-O-CH_2-O-$ | | $CO_2H$ | $-OCH_2-$ | 188 - 195 |
| 103a | H | H | H | $OC_{10}H_{21}$ | H | $CO_2H$ | $-OCH_2-$ | 227 - 250 |
| 104a | OMe | H | H | H | H | $CO_2Et$ | $-OCH_2-$ | 1720 |
| 105a | H | H | OMe | H | H | $CO_2Et$ | $-OCH_2-$ | 1725 |

| Bsp.-Nr. | $R^1$ | $R^2$ | $R^3$ | $R^4$ | $R^5$ | $R^6$ | A | Schmp. (°C) bzw. $\gamma_{CO}$ ($cm^{-1}$) |
|---|---|---|---|---|---|---|---|---|
| 106a | H | H | OMe | OMe | H | $CO_2Et$ | $-OCH_2-$ | 68 - 76 |
| 107a | H | H | OMe | H | OMe | $CO_2Et$ | $-OCH_2-$ | 1722 |
| 108a | OMe | OMe | H | H | H | $CO_2Et$ | $-OCH_2-$ | 1720 |
| 109a | H | H | OMe | OMe | OMe | $CO_2Et$ | $-OCH_2-$ | 1725 |
| 110a | $OCH(CH_3)_2$ | H | H | H | H | $CO_2Et$ | $-OCH_2-$ | 1718 |
| 111a | H | H | OPh | H | H | $CO_2Et$ | $-OCH_2-$ | 1722 |
| 112a | H | H | H | $O-2,4-Cl_2C_6H_3$ | H | $CO_2Et$ | $-OCH_2-$ | 78 - 91 |
| 113a | H | H | $OCH_2Ph$ | H | H | $CO_2Et$ | $-OCH_2-$ | 1725 |
| 114a | H | H | H | $-O-CH_2-O-$ | | $CO_2Et$ | $-OCH_2-$ | 1722 |
| 115a | H | H | H | $OC_{10}H_{21}$ | H | $CO_2Et$ | $-OCH_2-$ | 110 - 116 |
| 116a | $CH_3$ | H | H | H | H | $CO_2Et$ | $-CH_2O-$ | 1718 |
| 117a | H | H | H | $C(CH_3)_3$ | H | $CO_2Et$ | $-CH_2O-$ | 1720 |
| 118a | H | H | H | $C(CH_3)_2C_2H_5$ | H | $CO_2Et$ | $-CH_2O-$ | 35 - 40 |
| 119a | $CH_3$ | H | $CH_3$ | H | H | $CO_2Et$ | $-CH_2O-$ | 1720 |
| 120a | H | H | $CH_3$ | H | $CH_3$ | $CO_2Et$ | $-CH_2O-$ | 60 |
| 121a | $CH_3$ | $CH_3$ | H | $CH_3$ | H | $CO_2Et$ | $-CH_2O-$ | 80 - 83 |
| 122a | $CF_3$ | H | H | H | H | $CO_2Et$ | $-CH_2O-$ | 42 |
| 123a | H | H | H | -CH=CH-CH=CH- | | $CO_2Et$ | $-CH_2O-$ | 95 - 96 |
| 124a | J | H | H | H | H | $CO_2Et$ | $-CH_2O-$ | 48 - 50 |
| 125a | H | H | Cl | H | Cl | $CO_2Et$ | $-CH_2O-$ | 75 - 78 |
| 126a | Cl | Cl | H | H | H | $CO_2Et$ | $-CH_2O-$ | 75 - 80 |
| 127a | Cl | H | H | Cl | H | $CO_2Et$ | $-CH_2O-$ | 113 - 115 |
| 128a | H | Cl | H | $-O-CH_2-O-$ | | $CO_2Et$ | $-CH_2O-$ | 85 - 86 |

| Bsp.-Nr. | $R^1$ | $R^2$ | $R^3$ | $R^4$ | $R^5$ | $R^6$ | A | Schmp. (°C) bzw. $\Upsilon_{CO}$ ($cm^{-1}$) |
|---|---|---|---|---|---|---|---|---|
| 129a | H | H | H | OMe | H | $CO_2Et$ | $-CH_2O-$ | 73 - 75 |
| 130a | $CH_3$ | H | H | H | H | $CO_2H$ | $-CH_2O-$ | 168 - 171 |
| 131a | H | H | H | $C(CH_3)_3$ | H | $CO_2H$ | $-CH_2O-$ | > 200 |
| 132a | H | H | H | $C(CH_3)_2C_2H_5$ | H | $CO_2H$ | $-CH_2O-$ | 1675 |
| 133a | $CH_3$ | H | $CH_3$ | H | H | $CO_2H$ | $-CH_2O-$ | 170 - 175 |
| 134a | H | H | $CH_3$ | H | $CH_3$ | $CO_2H$ | $-CH_2O-$ | 170 - 172 |
| 135a | $CH_3$ | $CH_3$ | H | $CH_3$ | H | $CO_2H$ | $-CH_2O-$ | 1674 |
| 136a | $CF_3$ | H | H | H | H | $CO_2H$ | $-CH_2O-$ | 182 - 185 |
| 137a | J | H | H | H | H | $CO_2H$ | $-CH_2O-$ | 1668 |
| 138a | H | H | Cl | H | Cl | $CO_2H$ | $-CH_2O-$ | 1688 |
| 139a | Cl | Cl | H | H | H | $CO_2H$ | $-CH_2O-$ | 195 - 197 |
| 140a | Cl | H | H | Cl | H | $CO_2H$ | $-CH_2O-$ | 210 - 212 |
| 141a | H | Cl | H | $-O-CH_2-O-$ | | $CO_2H$ | $-CH_2O-$ | 1685 |
| 142a | H | H | H | OMe | H | $CO_2H$ | $-CH_2O-$ | 203 - 205 |
| 143a | H | H | H | $-C(CH_3)_2CH_2C(CH_3)[CH_2C(CH_3)_3]-$ | | $CO_2Et$ | $-CH_2O-$ | 1715 |
| 144a | H | H | H | $-C(CH_3)_2CH_2CH(CH_3)C(CH_3)_2-$ | | $CO_2Et$ | $-CH_2O-$ | 90 - 92 |
| 145a | H | H | H | $-C(CH_3)_2CH(CH_3)C(CH_3)_2-$ | | $CO_2Et$ | $-CH_2O-$ | 88 - 90 |
| 146a | $C(CH_3)_3$ | H | H | $C(CH_3)_3$ | OMe | $CO_2Et$ | $-CH_2O-$ | 1715 |
| 147a | H | H | H | $-C(CH_3)_2CH_2C(CH_3)[CH_2C(CH_3)_3]-$ | | $CO_2H$ | $-CH_2O-$ | 151 - 154 |
| 148a | H | H | H | $-C(CH_3)_2CH_2CH(CH_3)C(CH_3)_2-$ | | $CO_2H$ | $-CH_2O-$ | 1681 |
| 149a | H | H | H | $-C(CH_3)_2CH(CH_3)C(CH_3)_2-$ | | $CO_2H$ | $-CH_2O-$ | 1681 |
| 150a | $C(CH_3)_3$ | H | H | $C(CH_3)_3$ | OMe | $CO_2H$ | $-CH_2O-$ | 173 |
| 151a | H | $-CH=CH-CH=CH-$ | | H | H | $CO_2Et$ | $-CH_2O-$ | 60 - 65 |

| Bsp.-Nr. | $R^1$ | $R^2$ | $R^3$ | $R^4$ | $R^5$ | $R^6$ | A | Schmp. (°C) bzw. $\Upsilon_{CO}$ ($cm^{-1}$) |
|---|---|---|---|---|---|---|---|---|
| 152a | $CH_3$ | -CH=CH-CH=CH- | | H | H | $CO_2Et$ | $-CH_2O-$ | 133 |
| 153a | $CH_3$ | -CH=CH-CH=CH- | | H | H | $CO_2H$ | $-CH_2O-$ | 175 - 178 |
| 154a | H | -CH=CH-CH=CH- | | H | H | $CO_2Et$ | $-OCH_2-$ | 57 - 60 |
| 155a | H | -CH=CH-CH=CH- | | H | H | $CO_2H$ | $-OCH_2-$ | 200 - 202 |

**Tabelle 1b**

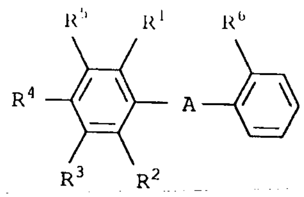

Ib

| Bsp.-Nr. | $R^1$ | $R^2$ | $R^3$ | $R^4$ | $R^5$ | $R^6$ | A | Schmp. (°C) bzw. $\Upsilon_{CO}$ ($cm^{-1}$) |
|---|---|---|---|---|---|---|---|---|
| 1b | $CH_3$ | H | H | H | H | $CO_2H$ | $-OCH_2-$ | 148 - 154 |
| 2b | $CH_3$ | H | $CH_3$ | H | H | $CO_2Me$ | $-OCH_2-$ | 68 - 70 |
| 3b | H | H | $CH_3$ | H | $CH_3$ | $CO_2Me$ | $-OCH_2-$ | 59 - 61 |
| 4b | $CH_3$ | $CH_3$ | $CH_3$ | H | H | $CO_2Me$ | $-OCH_2-$ | 83 - 85 |
| 5b | $CH_3$ | $CH_3$ | H | $CH_3$ | H | $CO_2Me$ | $-OCH_2-$ | 89 - 91 |
| 6b | H | H | H | $CH(CH_3)_2$ | H | $CO_2Me$ | $-OCH_2-$ | 1718 |
| 7b | H | H | Cl | Cl | H | $CO_2Me$ | $-OCH_2-$ | 70 - 72 |
| 8b | H | H | Ph | H | H | $CO_2Me$ | $-OCH_2-$ | 1720 |
| 9b | F | F | F | F | F | $CO_2Me$ | $-OCH_2-$ | 65 - 67 |
| 10b | $CH_3$ | H | H | $C(CH_3)_3$ | H | $CO_2Me$ | $-OCH_2-$ | 1720 |
| 11b | H | H | Cl | H | Cl | $CO_2Me$ | $-OCH_2-$ | 82 - 84 |
| 12b | H | H | H | $-CH_2-CH_2-CH_2-$ | | $CO_2Me$ | $-OCH_2-$ | 56 - 57 |
| 13b | H | H | H | $-CH_2-CH_2-CH_2-CH_2-$ | | $CO_2Me$ | $-OCH_2-$ | 59 - 62 |
| 14b | H | $-CH=CH-CH=CH-$ | | H | H | $CO_2Me$ | $-OCH_2-$ | 79 - 80 |
| 15b | H | H | H | $-C(CH_3)_2CH_2CH_2C(CH_3)_2-$ | | $CO_2Me$ | $-OCH_2-$ | 84 - 86 |
| 16b | $CH_3$ | H | $CH_3$ | H | H | $CO_2H$ | $-OCH_2-$ | 164 - 170 |
| 17b | H | H | $CH_3$ | H | $CH_3$ | $CO_2H$ | $-OCH_2-$ | 147 - 150 |
| 18b | $CH_3$ | $CH_3$ | $CH_3$ | H | H | $CO_2H$ | $-OCH_2-$ | 177 - 179 |
| 19b | $CH_3$ | $CH_3$ | H | $CH_3$ | H | $CO_2H$ | $-OCH_2-$ | 183 - 184 |

| Bsp.-Nr. | $R^1$ | $R^2$ | $R^3$ | $R^4$ | $R^5$ | $R^6$ | A | Schmp. (°C) bzw. $\gamma_{CO}$ ($cm^{-1}$) |
|---|---|---|---|---|---|---|---|---|
| 20b | H | H | H | $CH(CH_3)_2$ | H | $CO_2H$ | $-OCH_2-$ | 115 - 117 |
| 21b | H | H | Cl | Cl | H | $CO_2H$ | $-OCH_2-$ | 189 - 190 |
| 22b | H | H | Cl | H | Cl | $CO_2H$ | $-OCH_2-$ | 184 - 187 |
| 23b | $CH_3$ | H | H | $C(CH_3)_3$ | H | $CO_2H$ | $-OCH_2-$ | 157 - 171 |
| 24b | H | H | Phenyl | H | H | $CO_2H$ | $-OCH_2-$ | 111 - 175 |
| 25b | H | H | H | $-CH_2-CH_2-CH_2-$ | | $CO_2H$ | $-OCH_2-$ | 127 - 130 |
| 26b | H | H | H | $-CH_2-CH_2-CH_2-CH_2-$ | | $CO_2H$ | $-OCH_2-$ | 135 - 138 |
| 27b | H | -CH=CH-CH=CH- | | H | H | $CO_2H$ | $-OCH_2-$ | 169 - 172 |
| 28b | H | H | H | $-C(CH_3)_2CH_2CH_2C(CH_3)_2-$ | | $CO_2H$ | $-OCH_2-$ | 161 - 163 |
| 29b | $CH_3$ | H | $CH_3$ | H | H | $CO_2Me$ | $-CH_2O-$ | 49 - 51 |
| 30b | $CH_3$ | $CH_3$ | H | $CH_3$ | H | $CO_2Me$ | $-CH_2O-$ | 76 - 79 |
| 31b | H | H | H | $C(CH_3)_3$ | H | $CO_2Me$ | $-CH_2O-$ | 1729 |
| 32b | H | H | H | $C(CH_3)_2C_2H_5$ | H | $CO_2Me$ | $-CH_2O-$ | 1729 |
| 33b | H | H | Cl | Cl | H | $CO_2Me$ | $-CH_2O-$ | 61 - 64 |
| 34b | H | H | Cl | H | Cl | $CO_2Me$ | $-CH_2O-$ | 89 - 93 |
| 35b | $CH_3$ | H | $CH_3$ | H | H | $CO_2H$ | $-CH_2O-$ | 117 - 119 |
| 36b | $CH_3$ | $CH_3$ | H | $CH_3$ | H | $CO_2H$ | $-CH_2O-$ | 120 - 123 |
| 37b | H | H | H | $C(CH_3)_3$ | H | $CO_2H$ | $-CH_2O-$ | 142 - 146 |
| 38b | H | H | H | $C(CH_3)_2C_2H_5$ | H | $CO_2H$ | $-CH_2O-$ | 76 - 78 |
| 39b | H | -CH=CH-CH=CH- | | H | H | $CO_2H$ | $-CH_2O-$ | 101 - 102 |
| 40b | H | H | Cl | Cl | H | $CO_2H$ | $-CH_2O-$ | 129 - 132 |
| 41b | H | H | Cl | H | Cl | $CO_2H$ | $-CH_2O-$ | 137 - 140 |

Die erfindungsgemäß zu verwendenden Verbindungen bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten

in jedem Fall möglichst die feinste Verteilung der Wirkstoffe gewährleisten.

Normalerweise werden die Pflanzen mit den Wirkstoffen besprüht oder bestäubt oder die Samen der Pflanzen mit den Wirkstoffen behandelt.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Ligninsulfitablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Beispiele für solche Zubereitungen sind:

I. eine Lösung aus 90 Gew.-Teilen der Verbindung 2a und 10 Gew.-Teilen N-Methyl-a-pyrrolidon, die zur Anwendung in Form kleinster Tropfen geeignet ist;

II. eine Mischung aus 20 Gew.-Teilen der Verbindung 3a, 80 Gew.Teilen Xylol, 10 Gew.-Teilen des Anlagerungsproduktes von 8 bis 10 Mol Ethylenoxid an 1 Mol Ölsäure-N-monoethanolamid, 5 Gew.Teilen Calciumsalz der Dodecylbenzolsulfonsäure, 5 Gew.-Teilen des Anlagerungsproduktes von 40 Mol Ethylenoxid an 1 Mol Ricinusöl; durch feines Verteilen der Lösung in Wasser erhält man eine Dispersion.

III. eine wäßrige Dispersion aus 20 Gew.-Teilen der Verbindung 4a, 40 Gew.-Teilen Cyclohexanon, 30 Gew.-Teilen Isobutanol, 20 Gew.-Teilen des Anlagerungsproduktes von 40 mol Ethylenoxid an 1 mol Ricinusöl;

IV. eine wäßrige Dispersion aus 20 Gew.-Teilen der Verbindung 5a, 25 Gew.-Teilen Cyclohexanol, 65 Gew.-Teilen einer Mineralölfraktion vom Siedepunkt 210 bis 280`C und 10 Gew.-Teilen des Anlagerungsproduktes von 40 mol Ethylenoxid an 1 mol Ricinusöl;

V. eine in einer Hammermühle vermahlene Mischung aus 80 Gew.-Teilen der Verbindung 6a, 3 Gew.-Teilen des Natriumsalzes der Diisobutylnaphtalin-a-sulfonsäure, 10 Gew.-Teilen des Natriumsalzes einer Ligninsulfonsäure aus einer Sulfitablauge und 7 Gew.-Teilen pulverförmigem Kieselsäuregel; durch feines Verteilen der Mischung in Wasser erhält man eine Spritzbrühe;

VI. eine innige Mischung aus 3 Gew.-Teilen der Verbindung 7a und 97 Gew.-Teilen feinteiligem Kaolin; dieses Stäubemittel enthält 3 Gew.-% Wirkstoff;

VII. eine innige Mischung aus 30 Gew.-Teilen der Verbindung 8a, 92 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;

VIII. eine stabile wäßrige Dispersion aus 40 Gew.-Teilen der Verbindung 9a, 10 Gew.-Teilen des Natriumsalzes eines Phenolsulfonsäure-harnstoff-formaldehyd-Kondensates, 2 Gew.-Teilen Kieselgel und 48 Gew.-Teilen Wasser, die weiter verdünnt werden kann;

IX. eine stabile ölige Dispersion aus 20 Gew.-Teilen der Verbindung 10a, 2 Gew.-Teilen des Calciumsalzes der Dodecylbenzolsulfonsäure, 8 Gew.-Teilen Fettalkohol-polyglykolether, 20 Gew.-Teilen des Natriumsalzes eines Phenolsulfonsäure-harnstoff-formaldehyd-Kondensates und 68 Gew.-Teilen eines paraffinischen Mineralöls.

Die Verbindungen zeichnen sich durch eine hervorragende Wirksamkeit gegen ein breites Spektrum von pflanzenpathogenen Pilzen, insbesondere aus der Klasse der Phycomyceten, der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Die Verbindungen werden angewendet, indem man die Pilze oder die vor Pilzbefall zu schützenden Saatgüter, Pflanzen, Materialien oder den Erdboden mit einer fungizid wirksamen Menge der Wirkstoffe behandelt.

Die Anwendung erfolgt vor oder nach der Infektion der Materialien, Pflanzen oder Samen durch die Pilze.

Speziell eignen sich die Verbindungen I zur Bekämpfung folgender Pflanzenkrankheiten:
Helminthosporium-Arten an Getreide,
Septoria nodorum an Weizen,
Botrytis cinerea (Grauschimmel) an Erdbeeren, Reben,
Cercospora arachidicola an Erdnüssen,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die neuen Verbindungen können auch im Materialschutz (Holzschutz) eingesetzt werden, z.B. gegen Paecilomyces variotii.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff pro ha.

Bei der Saatgutbehandlung werden im allgemeinen Wirkstoffmengen von 0,001 bis 50 g, vorzugsweise 0,01 bis 10 g je Kilogramm Saatgut benötigt.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel,
Dithiocarbamate und deren Derivate, wie
Ferridimethyldithiocarbamat,
Zinkdimethyldithiocarbamat,
Zinkethylenbisdithiocarbamat,
Manganethylenbisdithiocarbamat,
Mangan-Zink-ethylendiamin-bis-dithiocarbamat,
Tetramethylthiuramdisulfide,
Ammoniak-Komplex von Zink-(N,N-ethylen-bis-dithiocarbamat),
Ammoniak-Komplex von Zink-(N,N'-propylen-bis-dithiocarbamat),
Zink-(N,N'-propylen-bis-dithiocarbamat),
N,N-Polypropylen-bis-(thiocarbamoyl)-disulfid;
Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat,
5-Nitro-isophthalsäure-di-isopropylester;
heterocyclische Substanzen, wie
2-Heptadecyl-2-imidazolin-acetat,

2,4-Dichlor-6-(o-chloranilino)-s-triazin,
O,O-Diethyl-phthalimidophosphonothioat,
5-Amino-1-βbis-(dimethylamino)-phosphinyl'-3-phenyl-1,2,4-triazol,
2,3-Dicyano-1,4-dithioanthrachinon,
2-Thio-1,3-dithioloβ4,5-b'chinoxalin,
1-(Butylcarbamoyl)-2-benzimidazol-carbaminsäuremethylester,
2-Methoxycarbonylamino-benzimidazol,
2-(Furyl-(2))-benzimidazol,
2-(Thiazolyl-(4))-benzimidazol,
N-(1,1,2,2-Tetrachlorethylthio)-tetrahydrophthalimid,
N-Trichlormethylthio-tetrahydrophthalimid,
N-Trichlormethylthio-phthalimid,
N-Dichlorfluormethylthio-N',N'-dimethyl-N-phenyl-schwefelsäurediamid,
5-Ethoxy-3-trichlormethyl-1,2,3-thiadiazol,
2-Rhodanmethylthiobenzthiazol,
1,4-Dichlor-2,5-dimethoxybenzol,
4-(2-Chlorphenylhydrazono)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin,
2,3-Dihydro-5-carboxanilido-6-methyl-1,4-oxathiin-4,4-dioxid,
2-Methyl-5,6-dihydro-4H-pyran-3-carbonsäure-anilid,
2-Methyl-furan-3-carbonsäureanilid,
2,5-Dimethyl-furan-3-carbonsäureanilid,
2,4,5-Trimethyl-furan-3-carbonsäureanilid,
2,5-Dimethyl-furan-3-carbonsäurecyclohexylamid,
N-Cyclohexyl-N-methoxy-2,5-dimethyl-furan-3-carbonsäureamid,
2-Methyl-benzoesäure-anilid,
2-Iod-benzoesäure-anilid,
N-Formyl-N-morpholin-2,2,2-trichlorethylacetal,
Piperazin-1,4-diylbis-(1-(2,2,2-trichlor-ethyl)-formamid,
1-(3,4-Dichloranilino)-1-formylamino-2,2,2-trichlorethan,
2,6-Dimethyl-N-tridecyl-morpholin bzw. dessen Salze,
2,6-Dimethyl-N-cyclododecyl-morpholin bzw. dessen Salze,
N-[3-(p-tert.-Butylphenyl)-2-methylpropyl]-cis-2,6-dimethylmorpholin,
N-[3-(p-tert.-Butylphenyl)-2-methylpropyl]-piperidin,
1-[2-(2,4-Dichlorphenyl)-4-ethyl-1,3-dioxolan-2-yl-ethyl]-1H-1,2,4-triazol
1-[2-(2,4-Dichlorphenyl)-4-n-propyl-1,3-dioxolan-2-yl-ethyl]-1H-1,2,4-triazol
N-(n-Propyl)-N-(2,4,6-trichlorphenoxyethyl)-N'-imidazol-yl-harnstoff,
1-(4-Chlorphenoxy)-3,3-dimethyl-1-(1H-1,2,4-triazol-1-yl)-2-butanon,
α-(2-Chlorphenyl)-α-(4-chlorphenyl)-5-pyrimidin-methanol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-83-methoxycarbonyl-2-thioureido)-benzol,
sowie verschiedene Fungizide, wie
Dodecylguanidinacetat,
3-[3-(3,5-Dimethyl-2-oxycyclohexyl)-2-hydroxyethyl)]glutarimid,
Hexachlorbenzol,
DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethylester,
5-Methyl-5-vinyl-3-(3,5-dichlorphenyl)-2,4-dioxo-1,3-oxazolidin,
3-[3,5-Dichlorphenyl(-5-methyl-5-methoxymethyl]-1,3-oxazolidin-2,4-dion,
3-(3,5-Dichlorhenyl)-1-isopropylcarbamoylhydantoin,
N-(3,5-Dichlorphenyl)-1,2-dimethylcyclopropan-1,2-dicarbonsäureimid,
2-Cyano-[N-(ethylaminocarbonyl)-2-methoximino]-acetamid,

1-[2-(2,4-Dichlorphenyl)-pentyl]-1H-1,2,4-triazol,
2,4-Difluor-α-(1H-1,2,4-triazolyl-1-methyl)-benzhydrylalkohol,
N-(3-Chlor-2,6-dinitro-4-trifluormethyl-phenyl)-5-trifluormethyl-3-chlor-2-aminopyridin,
1-((bis-(4-Fluorphenyl)-methylsilyl)-methyl)-1H-1,2,4-triazol.

Für die folgenden Versuche wurde als Vergleichswirkstoff 2-Cyan-N-(ethylaminocarbonyl)-2-methoximinoacetamid (A) - bekannt aus US-3 957 847 verwendet.

Anwendungsbeispiel
Wirksamkeit gegen Plasmopara viticola

Blätter von Topfreben der Sorte "Müller Thurgau" wurden mit wäßriger Spritzbrühe, die 80 % Wirkstoff und 20 % Emulgiermittel in der Trockensubstanz enthielt, besprüht. Um die Wirkungsdauer der Wirkstoffe beurteilen zu können, wurden die Pflanzen nach dem Antrocknen des Spritzbelages 8 Tage im Gewächshaus aufgestellt. Erst dann wurden die Blätter mit einer Zoosporenaufschwemmung von Plasmopara viticola (Rebenperonospora) infiziert. Danach wurden die Reben zunächst für 48 Stunden in einer wasserdampfgesättigten Kammer bei 24°C und anschließend für 5 Tage in einem Gewächshaus bei Temperaturen zwischen 20 und 30°C aufgestellt. Nach dieser Zeit wurden die Pflanzen zur Beschleunigung des Sporangienträgerausbruchs abermals für 16 Stunden in der feuchten Kammer aufgestellt. Danach erfolgte die Beurteilung des Ausmaßes des Pilzausbruchs auf den Blattunterseiten.

Das Ergebnis des Beispiels zeigt, daß die Verbindungen 1a, 14a, 16a, 18a und 132a bei der Anwendung als 60 ppm und 125 ppm Wirkstoff enthaltende Spritzbrühe eine bessere fungizide Wirkung zeigen (90 %) als der Vergleichswirkstoff A (15 %).

**Patentansprüche**

**1.** Verfahren zur Bekämpfung von Pilzen, dadurch gekennzeichnet, daß man eine fungizid wirksame Menge eines Diphenylheteroalkyl-Derivates der Formel I

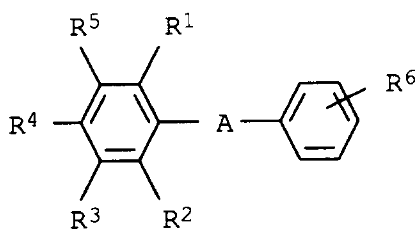

I

in der die Substituenten die folgenden Bedeutungen haben

A — eine $-X-CH_2-$, eine $-X-CH(CH_3)-$, eine $-CH_2-X-$ oder eine $-CH(CH_3)-X$-Gruppe, wobei X für ein Sauerstoffatom oder eine $S(O)_n$-Gruppe steht (mit n in der Bedeutung von 0, 1 oder 2)

$R^1,R^2,R^3,R^4,R^5$ — unabhängig voneinander Wasserstoff oder Halogen, eine $C_1$-$C_{12}$-Alkyl-, eine gegebenenfalls substituierte Phenyl-, eine $C_1$-$C_3$-Halogenalkyl- oder eine $OR^7$-Gruppe mit der Maßgabe, daß nur vier der Substituenten $R^1$, $R^2$, $R^3$, $R^4$ und $R^5$ Wasserstoff sein können

oder

$R^2$ und $R^3$ — gemeinsam unter Ausbildung eines Ringes eine $-CH=CH-CH=CH$-Gruppe

oder

$R^4$ und $R^5$ — gemeinsam unter Ausbildung eines Ringes eine $-CH_2-B-CH_2$-Gruppe [mit B in der Bedeutung $-CH_2-$ oder $-(CH_2)_2-$], eine $-C(CH_3)(CH_2R^8)-D-\ C(CH_3)_2$-Gruppe [mit D in der Bedeutung von $-(CH_2)_2-$, $-CH_2-CH(CH_3)-$ oder $-CHR^9-$], eine $-O-C(CH_3)(Z)-CH_2-CH_2$-Gruppe [mit Z = Methyl oder Ethyl, die jeweils durch den Rest $OR^{10}$ substituiert sein können], eine $-CH=CH-CH=CH$-Gruppe oder eine $-O-CH_2-O$-Gruppe,

$R^6$ — eine $COOR^{11}$-Gruppe, die in o-, m- oder p-Position zu A stehen kann,

$R^7$ — Wasserstoff, eine $C_1$-$C_{12}$-Alkyl-, eine $C_3$-$C_6$-Alkenyl-, eine $C_3$-$C_6$-Alkinyl-, eine $C_1$-$C_6$-Alkanoyl-, eine gegebenenfalls substituierte Benzyl- oder eine gegebenenfalls substituierte Phenylgruppe,

$R^8$ Wasserstoff oder eine $C_1$-$C_6$-Alkylgruppe,
$R^9$ Wasserstoff oder eine $C_1$-$C_4$-Alkylgruppe,
$R^{10}$ Wasserstoff, eine $C_1$-$C_6$-Alkyl- oder eine $C_1$-$C_6$-Alkanoylgruppe,
$R^{11}$ Wasserstoff oder eine $C_1$-$C_4$-Alkylgruppe,

oder dessen pflanzenverträglichen Salzes auf Pilze, vom Pilzbefall bedrohte Pflanzen, deren Lebensraum, deren Saatgut oder auf Materialien einwirken läßt.

**2.** Verfahren nach Anspruch 1, dadurch gekennzeichnet, daß man ein Diphenylheteroalkylderivat der Formel I verwendet, in der $R^6$ in para-Position zu A steht.

**3.** Verfahren nach Anspruch 1, dadurch gekennzeichnet, daß man ein Diphenylheteroalkylderivat der Formel I verwendet, in der $R^6$ in ortho-Position zu A steht.

Europäisches Patentamt

# EUROPÄISCHER RECHERCHENBERICHT

Nummer der Anmeldung

EP 92 12 1143

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| D,A | DE-A-2 252 818 (NIPPON SODA CO. LTD.)<br>--- | | A01N37/38<br>A01N37/40<br>A01N41/10<br>A01N43/16<br>A01N43/30 |
| D,A | CHEMICAL ABSTRACTS, vol. 98, no. 9, 28. Februar 1983, Columbus, Ohio, US; abstract no. 71705t, HODOGAYA CHEMICAL CO., LTD. 'm-(Benzyloxy)benzamides' Seite 592 ; * Zusammenfassung * & JP-A-57 149 254 (HODOGAYA CHEMICAL CO., LTD.)<br>--- | | |
| A | EP-A-0 472 224 (IMPERIAL CHEMICAL INDUSTRIES PLC)<br>----- | | |
| | | | RECHERCHIERTE SACHGEBIETE (Int. Cl.5 ) |
| | | | A01N |

Der vorliegende Recherchenbericht wurde für alle Patentansprüche erstellt

| Recherchenort | Abschlußdatum der Recherche | Prüfer |
|---|---|---|
| DEN HAAG | 23 MAERZ 1993 | DONOVAN T.M. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)